# EUROPEAN PATENT APPLICATION

(11) **EP 4 424 289 A1**
(43) Date of publication of application: **04.09.2024**
(21) Application number: 22886534.1
(22) Date of filing: 21.09.2022
(51) Int. Cl.: A61F 13/47, A61F 13/532, A61F 13/533

(54) **ABSORBENT ARTICLE**

(30) Priority: 28.10.2021 JP 2021176393
(71) Applicant: DAIO PAPER CORPORATION, Shikokuchuo-shi Ehime 799-0492 (JP)
(72) Inventor: KURIHARA, Ryoko, Sakura-shi, Tochigi 329-1411 (JP)
(74) Representative: Epping - Hermann - Fischer
(86) International application number: PCT/JP2022/035157
(87) International publication number: WO 2023/074201

(57) **Abstract**

An absorbent article includes an anti-slip portion at a side where a back sheet is disposed, and one or more compressed grooves formed at a side where a top sheet is disposed. The one or more compressed grooves include a pair of a left compressed groove and a right compressed groove, both extending along the front-back direction to cross over a body-fluid-discharging-orifice counter portion in the front-back direction, where the body-fluid-discharging-orifice counter portion is an area of the absorbent article facing a body-fluid discharging orifice of the user when the user wears the absorbent article. The left compressed groove, as a whole, has a shape that curves towards right to pass through a central region of the left-right direction. The right compressed groove, as a whole, has a shape that curves towards left to pass through the central region of the left-right direction. In a planar view of the absorbent article, the left compressed groove and the right compressed groove do not overlap the anti-slip portion in the central region of the left-right direction, and a front end and a back end of the left compressed groove, and a front end and a back end of the right compressed groove overlap the anti-slip portion.

## Description

### TECHNICAL FIELD

The present disclosure relates to an absorbent article.

### BACKGROUND ART

Absorbent articles of pad forms, such as incontinence pads, sanitary napkins, diaper pads, and the like, have been known. Structurally, such an absorbent article typically includes a liquid-permeable top sheet, a liquid-impermeable back sheet, an absorber disposed between the top sheet and the back sheet, and an anti-slip portion, which is configured to prevent slipping of the absorbent article from underwear, disposed on the back sheet side.

When a user wears an absorbent article, the absorbent article is three-dimensionally deformed to fit a body of the user. In the deformed state, the absorbent article further receives a force from legs of the user, and some areas of the anti-slip portion of the absorbent article are likely to detach from the underwear, if the user makes a large movement with the body, or the like. Therefore, an arrangement of an anti-slip portion considering a deformed state of an absorbent article when the absorbent article is worn has been studied, for example, regarding a relationship between an arrangement of an anti-slip portion and an arrangement of compressed grooves (fit embossings) that facilitate deformation of the absorbent article.

Patent Document 1 discloses an absorbent article including an anti-slip adhesive layer formed in a region that does not overlap fit embossings relative to a thickness direction of the absorbent article, where the anti-slip adhesive layer includes an adhesive portion for a groin area disposed at the side outer than the fit embossings relative to the width direction, and front and back adhesive portions that are formed at a front and back of the adhesive portion for the groin area to be spaced apart from each other. Patent Document 1 discloses that, when pressure from legs of a user is applied to the absorbent article in the worn state, adhesion between the sections of the anti-slip adhesive layer caused by shrinkage or deformation of the absorber facilitated by fit embossings can be prevented, and a problem such that adhesion between the anti-slip portions and underwear may not be achieved can be solved.

### RELATED-ART DOCUMENTS

### PATENT DOCUMENTS

Patent Document 1: Japanese Unexamined Patent Application Publication No. 2017-42471

### SUMMARY OF THE INVENTION

### PROBLEM TO BE SOLVED BY THE INVENTION

However, a desired deformed state (a shape after deformation) of an absorbent article when the absorbent article is worn varies depending on an intended use of the absorbent article, thus shapes of compressed grooves (fit embossings) for inducing the desired deformation vary. Therefore, an anti-slip portion (an anti-slip adhesive layer) may be detached from underwear depending on the shapes of the compressed grooves, and the detachment of the anti-slip portion may cause twisting of the absorbent article, leading to leakage of a body fluid.

Considering the above-described points, one aspect of the present invention aims to minimize detachment of a deformed absorbent article from underwear to prevent twisting of the absorbent article.

### MEANS TO SOLVE THE PROBLEM

According to one aspect of the present invention, an absorbent article includes a liquid-permeable top sheet, a liquid-impermeable back sheet, and an absorber disposed between the top sheet and the back sheet. The absorbent article has a front-back direction corresponding to a direction between a front and back of a body of a user, when the user wears the absorbent article, and a left-right direction orthogonal to the front-back direction. An anti-slip portion is disposed at a side of the absorbent article where the back sheet is disposed. One or more compressed grooves are formed at a side of the absorbent article where the top sheet is disposed. The one or more compressed grooves include a pair of a left compressed groove and a right compressed groove, both extending along the front-back direction to cross over a body-fluid-discharging-orifice counter portion in the front-back direction, where the body-fluid-discharging-orifice counter portion is an area of the absorbent article facing a body-fluid discharging orifice of the user when the user wears the absorbent article. The left compressed groove, as a whole, has a shape that curves towards right to pass through a central region of the left-right direction. The right compressed groove, as a whole, has a shape that curves towards left to pass through the central region of the left-right direction. In a planar view of the absorbent article, the left compressed groove and the right compressed groove do not overlap the anti-slip portion in the central region of the left-right direction, and a front end and back end of the left compressed groove, and a front end and back end of the right compressed groove overlap the anti-slip portion.

### EFFECTS OF THE INVENTION

According to one aspect of the present invention, an absorbent article, which can be easily deformed into a desired shape when the absorbent article is worn, and reduces occurrences of wrinkling or twisting at undesired positions, can be provided.

### BRIEF DESCRIPTION OF THE DRAWINGS

[Fig. 1] Fig. 1 is a planar view of an absorbent article of one embodiment viewed from a skin side.
[Fig. 2] Fig. 2 is a cross-sectional view of Fig. 1 cut along the line I-I.
[Fig. 3] Fig. 3 is a cross-sectional view of Fig. 1 cut along the line II-II.
[Fig. 4] Fig. 4 is a cross-sectional view illustrating a deformed state of the absorbent article, corresponding to Fig. 2.
[Fig. 5] Fig. 5 is a view for describing a function of the absorbent article according to one embodiment.
[Fig. 6] Fig. 6 is a partially enlarged view of Fig. 1.
[Fig. 7] Fig. 7 is a view illustrating a modification example of an anti-slip portion.
[Fig. 8] Fig. 8 is a view illustrating another modification example of the anti-slip portion.
[Fig. 9] Fig. 9 is a view illustrating yet another modification example of the anti-slip portion.
[Fig. 10] Fig. 10 is a view illustrating a modification example of compressed grooves.
[Fig. 11] Fig. 11 is a view illustrating another modification example of the compressed grooves.
[Fig. 12] Fig. 12 is a view illustrating yet another modification example of the compressed grooves.
[Fig. 13] Fig. 13 is a view illustrating yet another modification example of the compressed grooves.

### MODE FOR CARRYING OUT THE INVENTION

Embodiments of the present invention will be described with reference to drawings hereinafter. In the following drawings, the same reference signs are assigned to the same or corresponding components, unless otherwise stated, and duplication of description of the same or corresponding components may be omitted.

### <Basic structure of absorbent article>

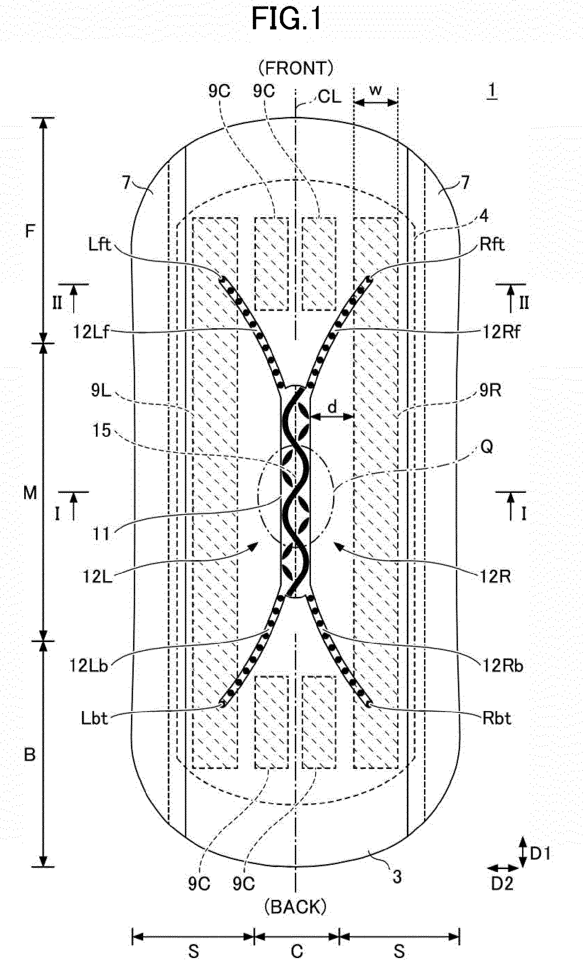

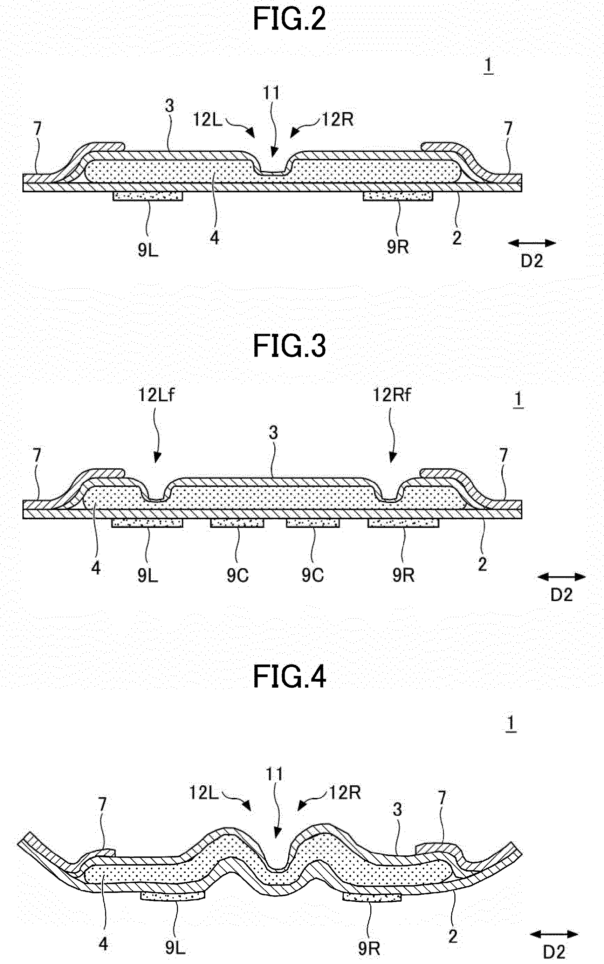

Fig. 1 depicts a planar view of the absorbent article 1 according to one embodiment of the present invention. The absorbent article of the illustrated embodiment is an incontinence pad. Fig. 2 is a cross-sectional view of the absorbent article 1 that is cut along the line I-I. Fig. 3 is a cross-sectional view of the absorbent article 1 that is cut along the line II-II. As illustrated in Figs. 1 to 3, the absorbent article 1 includes a liquid-permeable top sheet 3, a liquid-impermeable back sheet 2, and an absorber 4 between the top sheet and the back sheet, all of which are stacked. Before the absorbent article 1 is worn by a user, the absorbent article 1 as a whole has a substantially flat shape (Figs. 2 and 3). When the absorbent article 1 is worn, the side of the absorbent article 1 where the top sheet 3 is disposed is a side facing skin (skin side or front side), and the side of the absorbent article 1 where the back sheet 2 is disposed is a side fastened to underwear (underwear side or back side). Therefore, Fig. 1 is a view of the absorbent article 1 observed from the skin side.

In the absorbent article 1, a direction corresponding to a direction between a front and back of a body of a user, when the user wears the absorbent article 1, is determined as a front-back direction D1, and a direction orthogonal to the front-back direction D1 and corresponding to a direction between a left and right of the body of the user is determined as a left-right direction D2. In the present embodiment, the absorbent article 1 has a narrow and long shape so that the front-back direction D1 of the absorbent article 1 is a longitudinal direction, and the left-right direction D2 of the absorbent article 1 is a width direction. In the embodiment illustrated in Fig. 1, in a planar view of the absorbent article 1, the absorbent article 1 has a substantially line-symmetric shape with a center line (front-back direction center line) CL extending along the front-back direction D1 as a line of symmetry, but the shape of the absorbent article 1 may not be line-symmetry. Moreover, a structure (including a density of the absorber 4, materials, a size of the compressed groove, and the like) of the absorbent article 1 other than the shape thereof may be also substantially symmetric with the center line CL as a line of symmetry, or asymmetric.

The back sheet 2 may be a sheet having at least a water-shielding property. For example, the back sheet 2 may be a sheet formed of an olefin resin, such as polyethylene, polypropylene, and the like. Moreover, a laminate non-woven fabric, in which a non-woven fabric is laminated on a polyethylene sheet or the like, or a laminate sheet of non-woven fabrics where liquid impermeability is substantially imparted to the laminate sheet by inserting a waterproof film into the laminate sheet, or the like may be used. Moreover, more preferably, a sheet having moisture-permeability is used for reducing stuffiness. As a material of the above-described water-shielding and moisture-permeable sheet, a microporous sheet or the like may be used. The microporous sheet is obtained by melt-kneading an olefin-based resin, such as polyethylene, polypropylene, and the like, with an inorganic filler to shape a sheet, followed by stretching the sheet in a uniaxial or biaxial direction.

The top sheet 3 may be a sheet that can pass through body fluids, such as urine, menstrual blood, vaginal discharge, and the like. As the top sheet 3, a porous or non-porous non-woven fabric, a porous plastic sheet, or the like is suitably used. For a fibrous material constituting the non-woven fabric, one of, or a combination of two or more of synthetic fibers, such as olefin (e.g., polyethylene, polypropylene, and the like), polyester, polyamide, and the like, recycled fibers, such as rayon, cuprammonium rayon, and the like, blended fibers of the foregoing, or natural fibers, such as cotton and the like may be used. Examples of a processing method of the non-woven fabric include spun lacing, spun bonding, thermal bonding, melt blowing, needle punching, and the like. Among the above-listed processing methods, the spun lacing is preferred from the aspect of production of a non-woven fabric having excellent flexibility, spun bonding is preferred from the aspect of production of a non-woven fabric having excellent drapeability, and thermal bonding is preferred from the aspect of production of a bulky and soft non-woven fabric. Moreover, composite fibers, such as core-sheath composite fibers including a fiber having a high melting point as a core and a fiber having a low melting point as a sheath, side-by-side composite fibers, segmented-pie composite fibers, and the like, may be used.

The absorber 4 is not particularly limited, as long as the absorber 4 is a material that can absorb and retain a body fluid. The absorber 4 preferably includes cotton pulp and a water-absorbing polymer. As the water-absorbing polymer, a superabsorbent polymer (SAP) powder, a superabsorbent fiber (SAF), or a combination of the foregoing may be used. Examples of the pulp include chemical pulp obtained from wood, cellulose fibers, such as dissolving pulp, and pulp obtained from artificial cellulose fibers, such as rayon, acetate, and the like. As a raw material of the chemical pulp, hardwood, softwood, or the like may be used, but softwood is suitably used because hardwood has a long fiber length.

Synthetic fibers may be mixed with the absorber 4. As the synthetic fibers, polyolefin, such as polyethylene, polypropylene, and the like, polyester, such as polyethylene terephthalate, polybutylene terephthalate, and the like, polyamide, such as nylon and the like, or copolymers of the foregoing may be used. Among the above-listed examples, two or more of the listed examples may be used as a mixture. Moreover, composite fibers, such as core-sheath composite fibers including a fiber having a high melting point as a core and a fiber having a low melting point as a sheath, side-by-side composite fibers, segmented-pie composite fibers, and the like, may be also used. Hydrophobic fibers, which have been subjected to a surface treatment with a hydrophilizing agent to impart affinity to body fluids, may be used as the synthetic fibers. The absorber 4 is preferably produced by fiber-stacking or air laying.

The absorber 4 may be composed of a main body of the absorber 4 wrapped with a wrapping sheet formed of crepe paper, a non-woven fabric, or the like. Since the absorber 4 includes the wrapping sheet, twisting or breaking of the absorber 4 can be avoided, and a shape of the absorber 4 can be retained. As the wrapping sheet, crepe paper or a non-woven fabric that is not colored (i.e., white) may be used, or crepe paper or a non-woven fabric that is tinted in a color (e.g., tinted in a similar color to the color of a body fluid or tinted in a complementary color to the color of a body fluid) may be used.

As illustrated in Fig. 1, a planar shape of the absorber 4 may be a narrow and long shape having a substantially constant width. The width of the absorber 4 may change along the front-back direction D1, but the width of the absorber 4 is preferably substantially constant because a complex production process can be avoided. The absorber 4, as a whole, may have a uniform thickness across the whole area. The thickness of the absorber 4 may not be uniform, and the thickness of the absorber 4 may be partially thinner or thicker.

At the both ends of the absorber 4 in the front-back direction D1, an edge of the back sheet 2 and an edge of the top sheet 3 are bonded together with an adhesive, by heat-sealing, or the like. At the both sides of the absorbent article 1, i.e., the both sides of the left-right direction D2, a pair of side sheets 7 and 7 are disposed at the front side (the side where the top sheet 3 is disposed) along the front-back direction D1.

The side sheet 7 may be formed using a non-woven material which is subjected to suitable waterproof processing or hydrophilic processing according to the intended purpose, such as prevention of permeation of body fluids or improvement of the texture of the side sheet on skin. A material of the side sheet 7 may be natural fibers, synthetic fibers, recycled fibers, or the like. In the case where waterproof processing is performed on the side sheet 7, moreover, a waterproof agent, such as a silicone-based waterproof agent, a paraffin-based waterproof agent, and the like, may be used for the waterproof processing. The absorbent article may not use the side sheet 7 and may have a structure in which the top sheet 3 is extended to the edges of the absorbent article 1 in the left-right direction D2 to join the top sheet 3 and the back sheet 2.

At both sides of the absorbent article 1, gathers may be formed. The gathers are formed by enclosing a stretched state of an elastic member, such as a rubber string and the like, with a sheet. In this case, as the sheets enclosing the elastic members, the side sheets 7 and 7 may be used.

The absorbent article 1 may mainly include a middle region M corresponding to a groin area of a user when the user wears the absorbent article 1, a front region F that is next to the front of the middle region M and extends to the front end of the absorbent article 1, and a back region B that is next to the back of the middle region M and extends to the back end of the absorbent article 1. The middle region M includes a body-fluid-discharging-orifice counter portion Q. The body-fluid-discharging-orifice counter portion Q is a region facing a body-fluid discharging orifice of a user, such as an external urethral orifice, a vaginal orifice, and the like, when the user wears the absorbent article 1. A center of the body-fluid-discharging-orifice counter portion Q lies on the front-back direction center line CL. In the example illustrated in Fig. 1, the body-fluid-discharging-orifice counter portion Q is illustrated as an oval that is a region corresponding to an external urethral orifice of a user, but the size and shape of the illustrated body-fluid-discharging-orifice counter portion Q are merely an example for describing the absorbent article of the present embodiment.

When the absorbent article is individually packaged, the front region F and the back region B of the absorbent article may be folded over in the front-back direction D1 towards the skin side. In the example of Fig. 1, the front region F may be folded along a folding line that extends along a boundary between the front region F and the middle region M, and the back region B may be folded along a folding line that extends along a boundary between the back region B and the middle region M. As a result, the absorbent article 1 may be folded into three or more parts. It does not matter whichever region, the front region F or back region B, may be folded first.

Moreover, the absorbent article 1 may include a central region C and side regions S and S, relative to the left-right direction D2. The central region C includes the front-back direction center line CL. The side regions S and S are regions each of which is present next to the outer side of the central region C relative to the left-right direction D2 and extends to the outer edge of the absorbent article 1 in the left-right direction D2. The central region C may be a region that lies on the front-back direction center line CL and has a width that is approximately one-third of the width of the absorber 4.

A length (total length) of the entire absorbent article 1 along the front-back direction D1 may be preferably from 170 to 360 mm, and more preferably from 230 to 270 mm. A length of the absorbent article 1 along the left-right direction D2 (a width of a main body excluding wings in a case where the wings are provided) may be preferably from 50 to 150 mm, and more preferably from 70 to 120 mm. Moreover, a width of the absorber 4 disposed between the top sheet 3 and the back sheet 2 may be from 50 to 100 mm. Furthermore, a thickness of the entire absorbent article 1 may be preferably from 1 to 30 mm, and more preferably from 5 to 20 mm.

In the present embodiment, the absorbent article 1 does not include wings, but the absorbent article 1 may have a pair of wings extending out from the both side edges of the middle region M, respectively. Each wing may be formed by joining the extended portion of the side sheet 7 and the extended portion of the back sheet 2 together.

### <Anti-slip portion>

As illustrated in Figs. 1 to 3, an anti-slip portion may be formed at a side of the absorbent article 1 where the back sheet 2 is disposed. The anti-slip portion is configured to securely fasten the absorbent article 1 on underwear of a user to prevent slipping of the absorbent article 1 when the user wears the absorbent article 1. In the present embodiment, the anti-slip portion is preferably an adhesive anti-slip portion. The adhesive anti-slip portion is formed, for example, by applying an adhesive that is a fluid, or depositing a layer of an adhesive (e.g., an adhesive tape), which is formed in advance. As an adhesive used for the anti-slip portion, any adhesive available in the related art, such as a hot-melt adhesive, may be used. Examples of a main constituent component of the adhesive include styrene-based polymers, tackifiers, plasticizers, and a combination of the foregoing.

Examples of the above-mentioned styrene-based polymers include styrene-ethylene-butylene-styrene block copolymers, styrene-butylene-styrene block copolymers, styrene-isobutylene-styrene copolymers, styrene-butadiene-styrene block copolymers, and the like. One of the above-listed examples may be used alone, or two or more of the above-listed examples may be used in combination. Moreover, tackifiers and plasticizers that are solids at room temperature may be used as the above-mentioned tackifiers and plasticizers. Examples of the tackifiers include C5 petroleum resins, C9 petroleum resins, dicyclopentadiene-based petroleum resins, rosin-based petroleum resins, polyterpene resins, terpene phenolic resins, and the like. Examples of the plasticizers include monomeric plasticizers such as tricresyl phosphate, dibutyl phthalate, dioctyl phthalate, and the like, and additionally, polymeric plasticizers such as vinyl polymers, polyester, and the like.

As the anti-slip portion, one, or two or more anti-slip portions may be disposed. As illustrated in Fig. 1, the anti-slip portion includes at least a pair of a left anti-slip portion 9L and a right anti-slip portion 9R. The left anti-slip portion 9L and the right anti-slip portion 9R extend along the front-back direction D1 and are spaced apart from each other in the left-right direction D2. Since the left anti-slip portion 9L and the right anti-slip portion 9R are disposed in the side regions S and S of the left-right direction D2, respectively, the sides of the absorbent article 1 are more securely fastened to underwear along the front-back direction D1 so that a fastening function of the anti-slip portion can be effectively exhibited. Moreover, if the left anti-slip portion 9L and the right anti-slip portion 9R are disposed to stay within a region in which the absorber 4 is arranged in the planar view, as illustrated in Fig. 1, it is preferred because a thick part of the absorbent article where the absorber 4 is disposed is securely fastened onto underwear. Moreover, if the left anti-slip portion 9L and the right anti-slip portion 9R are each continuously formed along the front-back direction D1, it is preferred because a function of fastening the side regions S and S is enhanced to inhibit twisting or the like when a force is applied from both sides by legs.

A length (width) w of each of the left anti-slip portion 9L and the right anti-slip portion 9R along the left-right direction D2 may be from 5 to 20 mm (Fig. 1).

### <Compressed grooves>

As illustrated in Figs. 1 to 3, one or more compressed grooves that dip from the skin side to the non-skin side (i.e., dip from a side where the top sheet 3 is disposed to a side where the back sheet 2 is disposed) are formed in the absorbent article 1. The one or more compressed grooves may be one or more compressed grooves (fit embossings) formed by applying pressure to predetermined areas of a stack, in which at least the absorber 4 and the top sheet 3 are stacked, from the side where the top sheet 3 is disposed. The pressure is applied to correspond to predetermined shapes of the compressed grooves. For example, the compressed grooves as described above can be formed by passing a stack including the absorber 4 and the top sheet 3 between a roll having raised portions corresponding to predetermined shapes of the compressed grooves and a roll having no irregularities on a surface thereof facing the roll having the raised portions. Since the compressed grooves dip in the direction from the side where the top sheet 3 is disposed to the side where the back sheet 2 is disposed, as a force is applied to the absorbent article 1 from both sides of the left-right direction D2 by legs, the absorbent article is likely to be bent at positions where the compressed grooves are formed to curve towards the side where the back sheet 2 is disposed (the non-skin side or underwear side). In the case of the shapes of the compressed grooves of the present embodiment (the shapes where each of the left and right compressed grooves curves towards the front-back direction center line CL to pass through the central region C, the details will be described later), the absorbent article 1 is likely to deform in a manner such that the central region C of the left-right direction D2 sinks towards the underwear side in the middle region M of the absorbent article 1.

The degree of compression (the depth of the groove) within the compressed groove may be the same across the entire groove, or may be different in some areas of the groove. The compressed groove may include one or more low compression portions and one or more high compression portions that dip deeper than the low compression portions, where the high compression portions may be formed intermittently within the low compression portion. In the present specification, the low compression portions are represented in white and the high compression portions are represented in black (Fig. 1 etc.). Since the compressed grooves are each formed of a combination of the one or more high compression portions and the one or more low compression portions, the compressed grooves have a function of inducing deformation of the absorbent article, and the areas where the compressed grooves are formed are prevented from becoming excessively hard so that flexibility of the absorbent article can be assured.

As illustrated in Fig. 1, the compressed grooves may include a pair of compressed grooves, i.e., a left compressed groove 12L and a right compressed groove 12R, each extending along the front-back direction D1. In the present specification, the shape "extending along the front-back direction D1" means a shape which is recognized as following the front-back direction D1, not the left-right direction D2, as a whole, and may include, as well as a portion parallel to the front-back direction D1, a portion extending along a direction that forms an angle of 45° or less with the front-back direction D1. As illustrated in Fig. 1, the left compressed groove 12L and the right compressed groove 12R both extend to cross over the body-fluid-discharging-orifice counter portion Q in the front-back direction D1. Specifically, the range where the left compressed groove 12L and the right compressed groove 12R are disposed may be a range from the front region F to the back region B, passing through the middle region M. The left compressed groove 12L and the right compressed groove 12R may be each continuously or intermittently formed, but the left compressed groove 12L and the right compressed groove 12R are each preferably continuously formed. In the example of Fig. 1, the left compressed groove 12L is a portion that continues from the front end Lft to the back end Lbt, and the right compressed groove 12R is a portion that continues from the front end Rft to the back end Rbt, where the left compressed groove 12L and the right compressed groove 12R are joined together at a center of the left-right direction D2. Moreover, the joint of the left compressed groove 12L and the right compressed groove 12R may be linearly formed in the middle region M. In Fig. 1, the joint position 15 of the left compressed groove 12L and the right compressed groove 12R is represented with a dashed line.

As illustrated in Fig. 1, the left compressed groove 12L has a shape that curves towards the right, and the right compressed groove 12R has a shape that curves towards the left. In the example illustrated in Fig. 1, the left compressed groove 12L and the right compressed groove 12R have shapes that curve towards the front-back direction center line CL, respectively. Therefore, the gap between the left compressed groove 12L and the right compressed groove 12R becomes larger in the front region F, as the left compressed groove 12L and the right compressed groove 12R are getting closer to the front end of the absorbent article 1 in the front-back direction D1. Moreover, the gap between the left compressed groove 12L and the right compressed groove 12R becomes larger in the back region B, as the left compressed groove 12L and the right compressed groove 12R are getting closer to the back end of the absorbent article 1 in the front-back direction D1. Moreover, both the front end Lft of the left compressed groove 12L and the front end Rft of the right compressed groove 12R are disposed in the side regions S of the left-right direction D2, respectively, and both the back end Lbt of the left compressed groove 12L and the back end Rbt of the right compressed groove 12R are disposed in the side regions S of the left-right direction D2, respectively.

As described above, in the example of Fig. 1, the left compressed groove 12L and the right compressed groove 12R are joined together approximately at the center of the left-right direction D2 or in the central region C of the left-right direction D2, and approximately at the center of the front-back direction D1 or in the middle region M. More specifically, the left compressed groove 12L and the right compressed groove 12R are joined together within the central region C and within the middle region M. In the present example, moreover, a linear central compressed groove 11 is formed over the joined portion along the front-back direction D1. If the compressed grooves illustrated in Fig. 1 are described in another way, therefore, the compressed grooves include the linear central compressed groove 11 formed in the middle region M along the front-back direction D1, the front portion 12Lf of the left compressed groove L and the front portion 12Rf of the right compressed groove 12R, both extending from the central compressed groove 11 towards the front, and the back portion 12Lb of the left compressed groove 12L and the back portion 12Rb of the right compressed groove 12R, both extending from the central compressed groove 11 towards the back. It can be also described such that, within the compressed grooves illustrated in Fig. 1, a pair of the front portion 12Lf of the left compressed groove 12L and the front portion 12Rf of the right compressed groove 12R are branched from the front end of the central compressed groove 11, and a pair of the back portion 12Lb of the left compressed groove 12L and the back portion 12Rb of the right compressed groove 12R are branched from the back end of the central compressed groove 11.

A width (a length along the left-right direction D2) of the central compressed groove 11 is greater than each of widths of the front portion 12Lf and back portion 12Lb of the left compressed groove 12L and the front portion 12Rf and back portion 12Rb of the right compressed groove 12R. For example, the former may be 2 times or greater, and preferably 3 times to 5 times the latter.

As described above, the compressed grooves include the pair of the left compressed groove 12L and the right compressed groove 12R both extending along the front-back direction D1 to cross over the body-fluid-discharging-orifice counter portion Q in the front-back direction D1, where the left compressed groove 12L has a shape that curves towards the right to pass through the central region C and the right compressed groove 12R has a shape that curves towards the left to pass through the central region C. Therefore, the compressed grooves as a whole have a shape where a linear section (the central compressed groove 11) extending along the front-back direction D1 within the central region C of the left-right direction D2 in the middle region M, a section that is branched into two ways in a substantially V- or Y-shape (the front portion 12Lf of the left compressed groove 12L and the front portion 12Rf of the right compressed groove 12R) at the front of the linear section, and a section that is branched into two ways in a substantially V- or Y-shape (the back portion 12Lb of the left compressed groove 12L and the back portion 12Rb of the right compressed groove 12R) at the back of the linear section are continuous. As pressure is applied to both sides of the middle region M by legs when the absorbent article 1 is worn, the absorbent article 1 is therefore easily bent along the compressed grooves to induce three-dimensional deformation as a whole. As a result of the above-described facilitation of the deformation, desired deformation of the absorbent article is more assuredly achieved along the front-back direction D1 regardless of a manner how a user wears the absorbent article, or a body size of the user.

According to the above-described three-dimensional deformation, in the middle region M of the absorbent article 1, the central region C sinks towards the underwear side owing to the compressed groove section formed along the front-back direction D1 within the central region C of the left-right direction D2, thereby forming a dip extending along the front-back direction D1. Fig. 4 depicts a view corresponding to Fig. 2, and depicts a cross-sectional view of the middle region M of the absorbent article 1 in the deformed state. As illustrated in Fig. 4, the area of the absorbent article 1 where the left compressed groove 12L and the right compressed groove 12R are jointly formed (the area where the central compressed groove 11 is formed) sinks towards the side where the back sheet 2 is disposed, and the areas of the absorber 4 present on the left and right of the above-mentioned sunk area are deformed to be raised. Moreover, the front and back of the absorbent article 1 are lifted up towards the side where the top sheet 3 is disposed in comparison with the middle region M to fit a body shape of a user so that the front of the absorbent article 1 is brought into close contact with the surface near pubic bones at the front of the body, and the back of the absorbent article is brought into close contact with the round surfaces of the buttocks at the back of the body. In this case, because of the shape of the sections of the compressed groove branched into two ways, the front and back of the absorbent article 1 can be brought into close contact with a surface of a skin of a user without sinking the absorbent article 1 near the center of the left-right direction D2 towards the underwear side. When there is sudden discharge of a body fluid, or when a large amount of a body fluid having a low viscosity is discharged, therefore, the body fluid can be temporarily retained in the dip, and the body fluid can be rapidly diffused along the dip in the front-back direction D1. Moreover, at the front and back of the absorbent article 1, the face of the absorbent article 1 at the side where the top sheet 3 is disposed can be brought into close contact with the surface near pubic bones and the surfaces of the buttocks. Therefore, leakage in the front-back direction D1 can be prevented, and comfort at the front and back of the body can be improved when the absorbent article is worn.

Note that, in the example illustrated in Fig. 1, the front portion 12Lf of the left compressed groove 12L and the front portion 12Rf of the right compressed groove 12R are line-symmetrically formed with the front-back direction center line CL as a line of symmetry. Moreover, the back portion 12Lb of the left compressed groove 12L and the back portion 12Rb of the right compressed groove 12R are also line-symmetrically formed with the front-back direction center line CL as a line of symmetry. Moreover, the portions 12Lf, 12Rf, 12Lb, and 12Rb are all arc shapes that are convex towards the front-back direction center line CL (curving towards the front-back direction center line CL). Since the front portions 12Lf and 12Rf and the back portions 12Lb and 12Rb are curved in the above-described manner, a naturally deformed state of the absorbent article that fits the front and back of a body of a user can be obtained.

### <Relationship between compressed grooves and anti-slip portion>

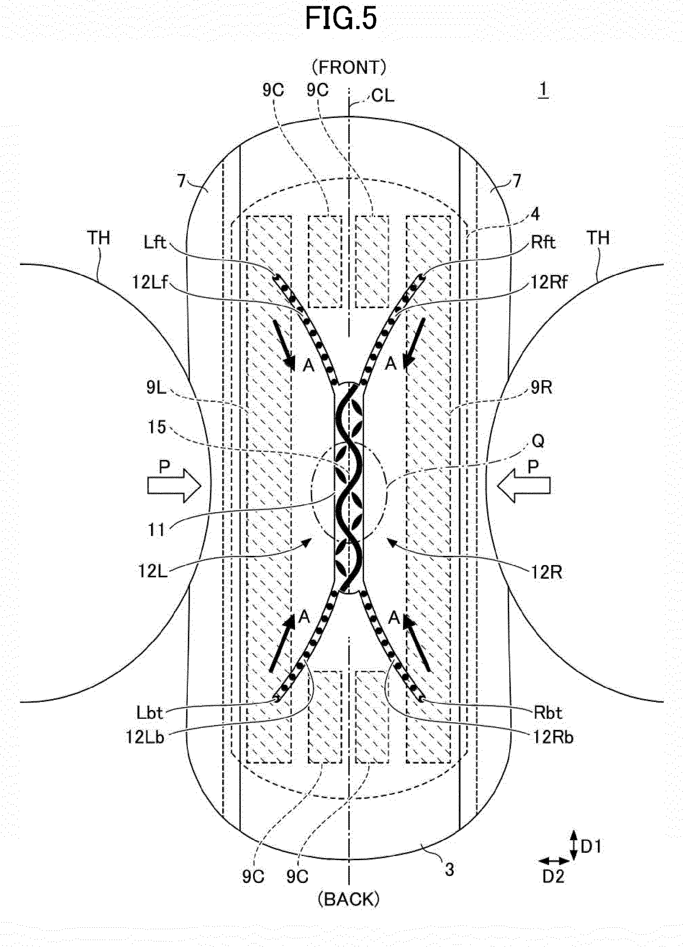

When the absorbent article 1 is set, the absorbent article 1 is adhered onto underwear using the above-described anti-slip portion. Then, as the absorbent article 1 is set on a body of a user, the absorbent article 1 is three-dimensionally deformed as described above to fit a shape of the body. When the absorbent article 1 is worn, the absorbent article 1 is preferably fastened onto the underwear while maintaining the deformed state. However, the absorbent article 1 may be distorted and detached from the underwear by large movements of legs of a user or the like. In this case, the absorbent article 1, which has been temporarily detached, may be fastened to the underwear again in an undesirable deformation state. Particularly with a structure of the absorbent article of the present embodiment including a pair of the left compressed groove 12L and the right compressed groove 12R having certain curve shapes, a force is likely to be applied on the front end Lft of the left compressed groove 12L and the front end Rft of the right compressed groove 12R of the absorbent article 1, the back end Lbt of the left compressed groove 12L and the back end Rbt of the right compressed groove 12R of the absorbent article 1, and surrounding areas of the foregoing, and the absorbent article 1 is likely to detach from the underwear. This point will be more specifically described with reference to a schematic view (Fig. 5).

Fig. 5 again depicts the planar view of the absorbent article 1 of Fig. 1, to which further description is added for explanation. As illustrated in Fig. 5, when the absorbent article 1 is worn, the sides of the middle region M of the absorbent article 1 receives the force P and P from legs TH and TH of a user in the direction from the outside to the inside relative to the left-right direction D2. When the force is applied, the side regions S at the front and back of the legs TH and TH are likely to be pulled in the direction towards the center of the front-back direction D1 and the center of the left-right direction D2 (arrows A of Fig. 5). Particularly, the front end Lft of the left compressed groove 12L and the front end Rft of the right compressed groove 12R, the back end Lbt of the left compressed groove 12L and the back end Rbt of the right compressed groove 12R, and the surroundings of the foregoing are portions that are intrinsically likely to be bent and distorted by the compressed grooves, and are therefore portions that could be readily detached from the underwear by application of external force. Therefore, detachment of (the anti-slip portion of) the absorbent article 1 from the underwear could potentially occur at the front ends Lft and Rft, and the back ends Lbt and Rbt of the compressed grooves.

In the present embodiment, considering the above, the front end Lft and back end Lbt of the left compressed groove 12L overlap the left anti-slip portion 9L, and the front end Rft and back end Rbt of the right compressed groove 12R overlap the right anti-slip portion 9R. Therefore, portions of the absorbent article 1 where the edges (front ends Lft and Rft, and back ends Lbt and Rbt) of the compressed grooves 12L and 12R extending to the four corners of the absorbent article 1 are present, and surrounding portions of the foregoing are more securely fastened onto underwear. As a result, the sliding of the entire absorbent article 1 from the underwear, or change of the deformed shape of the absorbent article 1 to an unsuitable shape can be prevented.

As illustrated in Figs. 1 to 5, moreover, the left compressed groove 12L and the right compressed groove 12R do not overlap the anti-slip portion in the central region of the left-right direction D2. Moreover, the anti-slip portion does not overlap the linear central compressed groove 11 formed by joining the left compressed groove 12L and the right compressed groove 12R. The compressed groove extending within the central region C of the left-right direction D2 is formed in the middle region M. When the absorbent article 1 is worn, the middle region M receives the force P and P from both legs TH and TH to reduce the width of the absorbent article 1 (Fig. 5). Therefore, it is likely that the anti-slip portions come into contact with each other to bond together in the left-right direction D2 at the side of the absorbent article 1 where the back sheet 2 is disposed. Since an anti-slip portion is not formed in the central region C of the left-right direction D2 where the compressed grooves extend, probability of adhesion between the anti-slip portions can be therefore reduced.

In the middle region M, therefore, another anti-slip portion is preferably not disposed between the left anti-slip portion 9L and right anti-slip portion 9R extending along the front-back direction D1.

In the middle region M, moreover, the left compressed groove 12L or right compressed groove 12R and the anti-slip portion are preferably spaced apart from each other in the left-right direction D2. The maximum separation distance between the left compressed groove 12L or right compressed groove 12R and the anti-slip portion may be preferably from 5 to 30 mm, and more preferably from 8 to 20 mm. In the example illustrated in Fig. 1, the separation distance is represented by a separation distance d between the linear central compressed groove 11 and the right anti-slip portion 9R in the left-right direction D2. Moreover, the separation distance d is preferably from 35 to 50% of the length between the edge of one side of the central compressed groove 11 in the left-right direction D2 and the edge of one side of the absorber 4 that is on the same side as the edge of the central compressed groove 11 in the left-right direction D2. Since the left anti-slip portion 9L and the right anti-slip portion 9R are each spaced apart from the central compressed groove 11 within the above-mentioned range, a flexible region can be secured between the central compressed groove 11 and the anti-slip portion 9L or 9R. In the case where the absorbent article 1 receives a force from the left and the right by legs of a user when the user wears the absorbent article 1, therefore, the section between the central compressed groove 11 and the left anti-slip portion 9L and the section between the central compressed groove 11 and the right anti-slip portion 9R can be raised to the skin side as viewed from the left-right direction D2 so that dipping of the central compressed groove 11 can be further facilitated (Fig. 4).

In the case where the central compressed groove 11 is formed, a width w of the anti-slip portion (a length of the left anti-slip portion 9L or right anti-slip portion 9R along the left-right direction D2) may be from 35 to 50% of the width between the edge of one side of the central compressed groove 11 in the left-right direction D2 and the edge of one side of the absorber 4 that is on the same side as the edge of the central compressed groove 11 in the left-right direction D2.

Note that, in the front region F and the back region B, an anti-slip portion may be disposed between the left anti-slip portion 9L and right anti-slip portion 9R extending along the front-back direction D1. As illustrated in Fig. 1, for example, central anti-slip portions 9C and 9C may be formed. The central anti-slip portions 9C and 9C are formed in the front region F and/or back region B, and are preferably not formed in the middle region M. Moreover, the central anti-slip portions 9C and 9C may extend to the middle region M, but preferably do not overlap the central compressed groove 11.

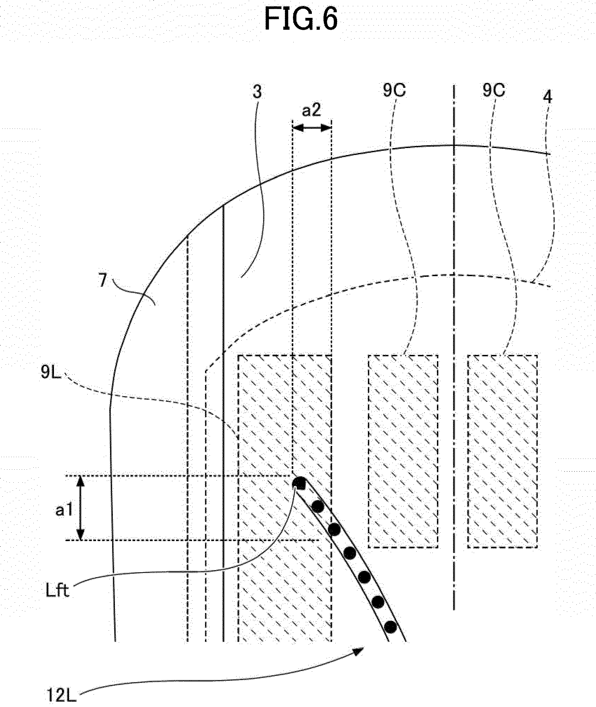

Fig. 6 depicts a partially enlarged view of Fig. 1. Fig. 6 illustrates an enlarged view of the area near the front end Lft of the left compressed groove 12L. As described above, in the present embodiment, the front end Lft and back end Lbt of the left compressed groove 12L overlap the left anti-slip portion 9L, and the front end Rft and back end Rbt of the right compressed groove 12R overlap the right anti-slip portion 9R so that the absorbent article 1 is stably fastened onto underwear and a desired deformation induced by the compressed grooves can be retained when the absorbent article 1 is worn. The edge of the compressed groove and the anti-slip portion preferably overlap in the front-back direction D1 and in the left-right direction D2. As illustrated in Fig. 6, the length a1 of the portion where the front end Lft of the left compressed groove 12L and the left anti-slip portion 9L overlap in the front-back direction D1 may be preferably 3 mm or greater, and more preferably 5 mm or greater. Moreover, the length a2 of the portion where the front end Lft of the left compressed groove 12L and the left anti-slip portion 9L overlap in the left-right direction D2 may be preferably 3 mm or greater, and more preferably 5 mm or greater. Moreover, the area of the portion where the front end Lft of the left compressed groove 12L and the left anti-slip portion 9L overlap is preferably from approximately 5 to 25 mm². Since the compressed groove overlaps the anti-slip portion in the above-described range of the length and in the above-described range of the area in the planar view, even if pressure is applied from legs, detachment of the absorbent article 1 from underwear can be prevented when the absorbent article 1 is worn, and occurrences of wrinkling or twisting at an undesired position can be prevented.

The preferred ranges of the length and area in the above description with reference to Fig. 6 are the same for other edges of the compressed grooves that are not illustrated in Fig. 6, i.e., the back end Lbt of the left compressed groove 12L, and the front end Rft and back end Rbt of the right compressed groove 12R. The degree of overlapping (overlapped length and area) between the compressed groove and the anti-slip portion in the planar view may be different among the above-mentioned four ends of the compressed grooves. However, the degree of overlapping is preferably the same between the front ends Lft and Rft, and/or between the back ends Lbt and Rbt so that the left side and the right side are suitably balanced. Moreover, the degree of overlapping (overlapped length and area) between the compressed groove and the anti-slip portion in the planar view is preferably the same among all ends Lft, Rft, Lbt, and Rbt of the compressed grooves so that the resistance force of the absorbent article against detachment can be suitably balanced at the four corners of the absorbent article.

### <Modification examples of anti-slip portion>

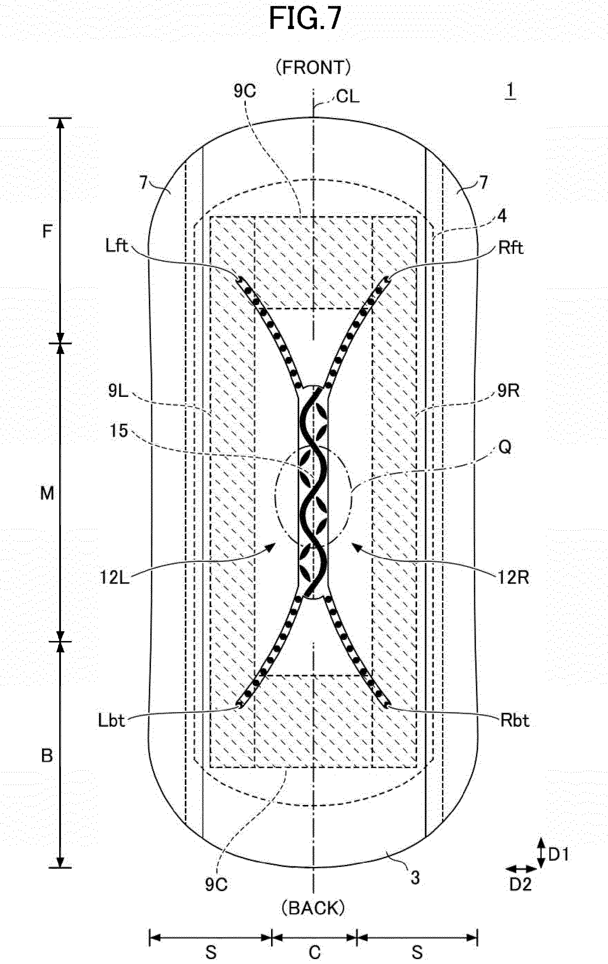

Figs. 7 to 9 depict absorbent articles each including an anti-slip portion according to a modification example. In the example illustrated in Fig. 7, the structure other than the anti-slip portion is identical to the structure illustrated in Fig. 1. The anti-slip portion illustrated in Fig. 7 includes a pair of a left anti-slip portion 9L and a right anti-slip portion 9R both extending along the front-back direction D1, where a central anti-slip portion 9C is disposed between the left anti-slip portion 9L and the right anti-slip portion 9R in the front region F, and the left anti-slip portion 9L, the central anti-slip portion 9C, and the right anti-slip portion 9R are continuously formed in the left-right direction D2. Moreover, a central anti-slip portion 9C is also disposed between the left anti-slip portion 9L and the right anti-slip portion 9R in the back region B, and the left anti-slip portion 9L, the central anti-slip portion 9C, and the right anti-slip portion 9R are continuously formed in the left-right direction D2. Because of the structure as described above, the connection between the anti-slip portions in the left-right direction is enhanced at the front and back of the absorbent article 1 so that a function of the anti-slip portions to fasten the absorbent article 1 onto underwear is improved.

In the example illustrated in Fig. 7, the front ends of the left anti-slip portion 9L, the central anti-slip portion 9C, and the right anti-slip portion 9R in the front region F are aligned, and the back ends of the left anti-slip portion 9L, the central anti-slip portion 9C, and the right anti-slip portion 9R in the back region B are aligned. Therefore, as a whole view, a continuous anti-slip portion in the shape of a rectangular annulus is formed. Because of the shape as described, fastening of the periphery of the absorbent article 1 including the front and back and the left and right of the absorbent article 1 to underwear is enhanced so that occurrences of undesirable partial wrinkling or twisting are prevented, and sliding of the entire absorbent article 1 can be also prevented.

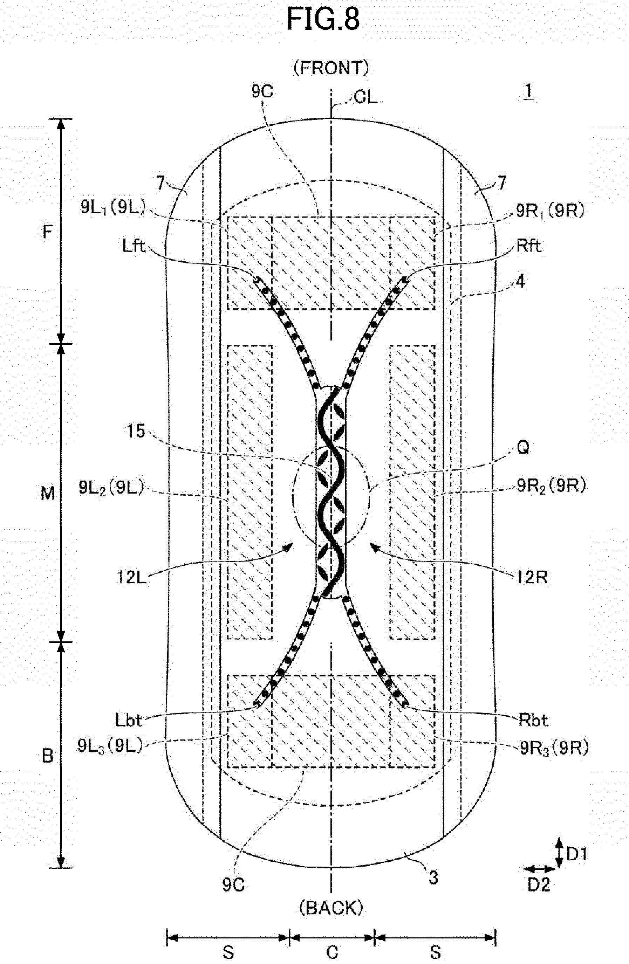

In the example illustrated in Fig. 8, the left anti-slip portion 9L and the right anti-slip portion 9R are both aligned along the front-back direction D1, but are intermittent in the front-back direction D1. Specifically, left anti-slip portion segments 9L₁, 9L₂, and 9L₃ that intermittently extend along the front-back direction D1, and right anti-slip portion segments 9R₁, 9R₂, and 9R₃ that intermittently extend along the front-back direction D1 are formed. The example of the anti-slip portion including the intermittent segments is preferred from the viewpoint of securing flexibility along the front-back direction D1. In each of the left anti-slip portion 9L and the right anti-slip portion 9R extending along the front-back direction D1, however, a length of the intermittent segment along the front-back direction D1 is preferably 20% or less of the total length of the entire left anti-slip portion 9L or the total length of the entire right anti-slip portion 9R.

Moreover, a central anti-slip portion 9C is disposed between the left anti-slip portion segment 9L₁ and the right anti-slip portion segment 9R₁ in the front region F, and a central anti-slip portion 9C is also disposed between the left anti-slip portion segment 9L₃ and the right anti-slip portion segment 9R₃ in the back region B. Moreover, in the front region F, the left anti-slip portion segment 9L₁, the central anti-slip portion 9C, and the right anti-slip portion segment 9R₁ are continuously formed in the left-right direction D2. Similarly, in the back region B, the left anti-slip portion segment 9L₃, the central anti-slip portion 9C, and the right anti-slip portion segment 9R₃ are continuously formed in the left-right direction D2. Because of the structure as described above, the connection between the anti-slip portions in the left-right direction D2 is enhanced at the front and back of the absorbent article 1 so that the front and back of the absorbent article 1 (near the front end and the back end of the absorber 4) can be stably fastened onto underwear, preventing undesirable twisting, wrinkling, or the like.

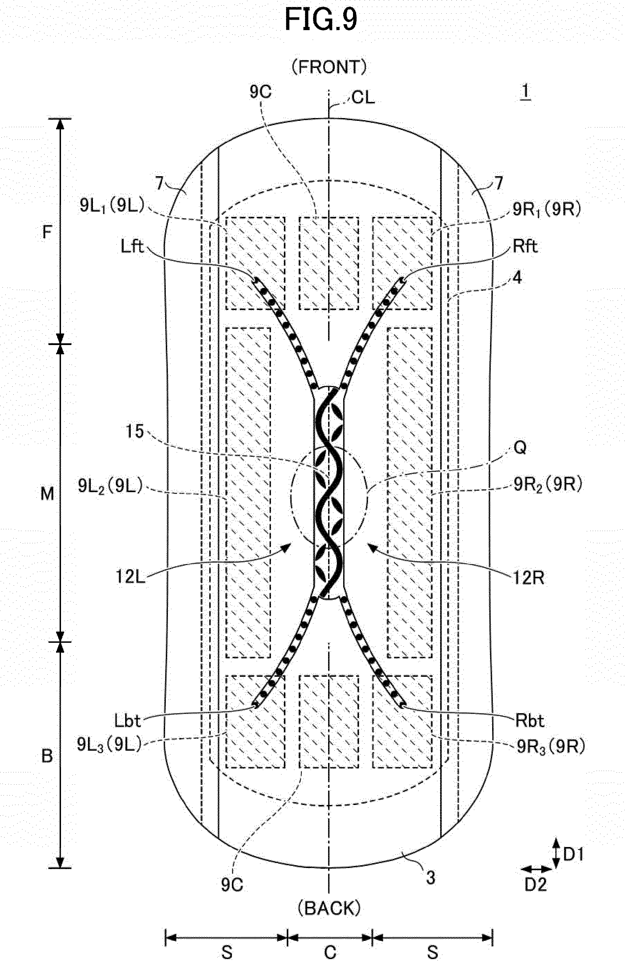

Similar to the example illustrated in Fig. 8, in the example illustrated in Fig. 9, a left anti-slip portion 9L and a right anti-slip portion 9R are each intermittent in the front-back direction D1, and the same effect can be obtained. In the example illustrated in Fig. 9, moreover, a central anti-slip portion 9C formed between a left anti-slip portion segment 9L₁ and a right anti-slip portion segment 9R₁ in the left-right direction D2 in the front region F is spaced apart from each of the left anti-slip portion segment 9L₁ and the right anti-slip portion segment 9R₁. In the back region B, moreover, a central anti-slip portion 9C formed between a left anti-slip portion segment 9L₃ and a right anti-slip portion segment 9R₃ in the left-right direction D2 is spaced apart from each of the left anti-slip portion segment 9L₃ and the right anti-slip portion segment 9Rs. Therefore, the example illustrated in Fig. 9 is preferred from the viewpoint of securing flexibility of the central region of both the front region F and the back region B, as well as securing flexibility of the side regions S and S.

Note that, in the example illustrated in Fig. 9, among the left anti-slip portion segments 9Li, 9L₂, and 9L₃ included in the left anti-slip portion 9L, each of the left anti-slip portion segment 9L₁ positioned at the front and the left anti-slip portion segment 9L₃ positioned at the back has the larger length (width) along the left-right direction D2 than the length of the left anti-slip portion segment 9L₂ positioned in the middle of the front-back direction D1. Similarly, among the right anti-slip portion segments 9R₁, 9R₂, and 9R₃ included in the right anti-slip portion 9R, each of the right anti-slip portion segment 9R₁ positioned at the front and the right anti-slip portion segment 9R₃ positioned at the back has the larger length (width) along the left-right direction D2 than the length of the right anti-slip portion segment 9R₂ positioned in the middle of the front-back direction D1. The left anti-slip portion segments 9L₁ and 9L₃ and the right anti-slip portion segments 9R₁ and 9R₃ all overlap with the edges (front ends or back ends) of the compressed grooves. Therefore, the left anti-slip portion segments 9L₁ and 9L₃ and the right anti-slip portion segments 9R₁ and 9R₃ each having a large width can stably fasten the regions near the edges of the compressed grooves to underwear.

As described above, in all the examples illustrated in Figs. 7 to 9, the arrangement of the anti-slip portion is different, but the front end Lft and back end Lbt of the left compressed groove 12L, and the front end Rft and back end Rbt of the right compressed groove 12R overlap the anti-slip portion in the regions of the four corners of the absorbent article 1 in the planar view. More specifically, all of the front ends and back ends of the pair of the compressed grooves disposed in the absorbent article 1 along the front-back direction D1 overlap the anti-slip portion in the both side regions S and S in the front region F and the back region B of the absorbent article 1. Because of the structure as mentioned above, the portions of the absorbent article 1 that are likely to be distorted are more securely fastened onto underwear when the absorbent article 1 is worn, and partial detachment of the absorbent article 1, which may cause occurrences of undesirable wrinkling, twisting, or the like at the detached portions, can be prevented. Therefore, the absorbent article 1 can retain a desirably deformed state so that a body fluid can be more assuredly absorbed.

### <Modification examples of compressed grooves>

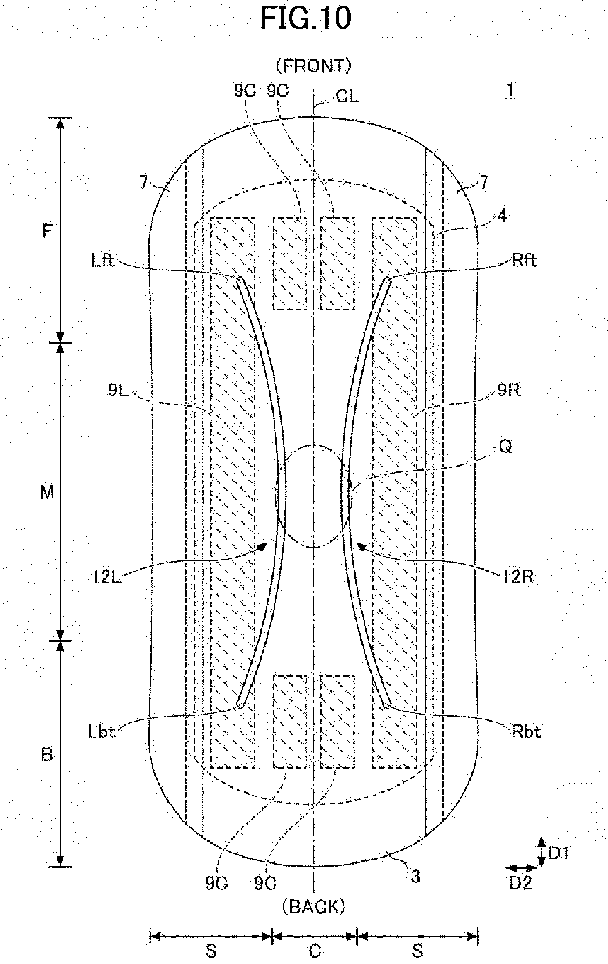

Figs. 10 to 13 depict absorbent articles 1 each including compressed grooves according to a modification example. In each of the examples illustrated in Figs. 10 to 13, similar to the example illustrated in Fig. 1, a left compressed groove 12L having a shape that curves towards the right to pass through the central region C of the left-right direction D2 in the middle region M, and a right compressed groove 12R having a shape that curves towards the left to pass through the central region C of the left-right direction D2 in the middle region M are formed. In other words, the left compressed groove 12L and right compressed groove 12R are formed in a manner such that the front ends and back ends thereof are arranged in the side regions S and S, and the left compressed groove 12L and right compressed groove 12R both have curve shapes that come close to each other near the center of the front-back direction D1, more specifically, in the middle region. Moreover, the compressed grooves illustrated in Figs. 10 to 13 may be also composed of a combination of high compression portions and low compression portions as illustrated in Fig. 1, but illustration of high compression portions is omitted in Figs. 10 to 13.

In the example illustrated in Fig. 10, the right compressed groove 12R and the left compressed groove 12L do not come into contact with each other, nor overlap each other, and are spaced apart from each other. As illustrated in Fig. 10, the position at which the right compressed groove 12R and the left compressed groove 12L are the closest to each other is within the body-fluid-discharging-orifice counter portion Q in the front-back direction D1. Also in the example of Fig. 10, as described with reference to the example of Fig. 1, if the both sides receive a force from legs when the absorbent article is worn, the absorbent article is deformed in a manner such that the central region C of the left-right direction D2 sinks towards the underwear side near the center of the front-back direction D1 (middle region M), and the front region F and the back region B are raised to the skin side to be in close contact with the surface of the skin of the user. Since the right compressed groove 12R and the left compressed groove 12L are spaced apart from each other in the example illustrated in Fig. 10, the width of the bottom of the groove-like dip in the central region C can be widened. Therefore, the example of Fig. 10 is preferred from the viewpoint of retention of a large amount of a body fluid, and also from the viewpoint of enhanced flexibility of the entire absorbent article 1.

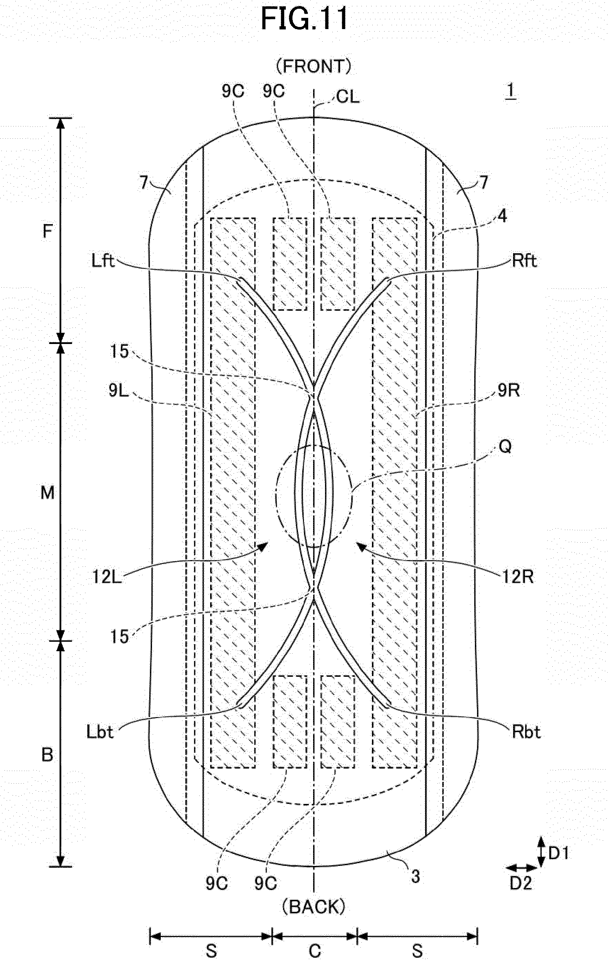

In the example illustrated in Fig. 11, the right compressed groove 12R and the left compressed groove 12L cross over each other. As rigidity is increased at crossing points, the crossing points can contribute to retention of the shape of the absorbent article that is curved in the state where the front portion and the back portion of the absorbent article are deformed to curve towards the skin side, when the absorbent article is worn. Similar to the example illustrated in Fig. 10, moreover, the example of Fig. 11 is preferred from the viewpoint that the width of the bottom of the groove-like dip formed in the central region C can be widened when deformed. Moreover, the region surrounded by the compressed grooves formed in the central region C becomes narrower, as the region comes closer to the front from the center of the front-back direction D1 and closer to the back from the center of the front-back direction D1, thus the region surrounded by the compressed grooves formed in the central region C can contribute to retain a body fluid within the region. Therefore, probability of leakage at the front and the back can be reduced when the body fluid moves in the front-back direction D1 along the groove-like dip.

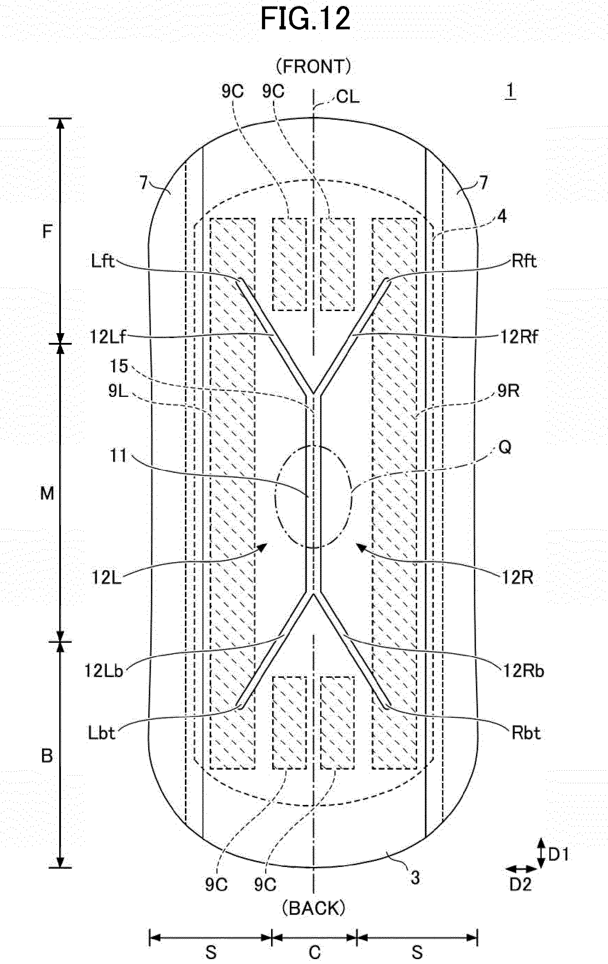

In the example illustrated in Fig. 12, the right compressed groove 12R and the left compressed groove 12L are each linearly formed, and are joined together near the center of the front-back direction D1 (in the middle region M) with a joint line 15 to form a linear central compressed groove 11 in the central region C. In the present example, therefore, similar to the example illustrated in Fig. 1, the compressed grooves have a shape that includes the central compressed groove 11, a pair of front portions 12Lf and 12Rf branched from the front end of the central compressed groove 11, and a pair of back portions 12Lb and 12Rb branched from the back end of the central compressed groove 11. The gap between the pair of the front portions 12Lf and 12Rf becomes larger, as the position of the gap is getting closer to the front end of the absorbent article 1. The gap between the pair of the back portions 12Lb and 12Rb becomes larger, as the position of the gap is getting closer to the back end of the absorbent article 1. Therefore, the region between the pair of the front portions 12Lf and 12Rf can be brought into close contact with the area near pubic bones at the front of the body, and the region between the pair of the back portions 12Lb and 12Rb can be brought into close contact with the curves of the buttocks so that leakage can be prevented at the front and the back.

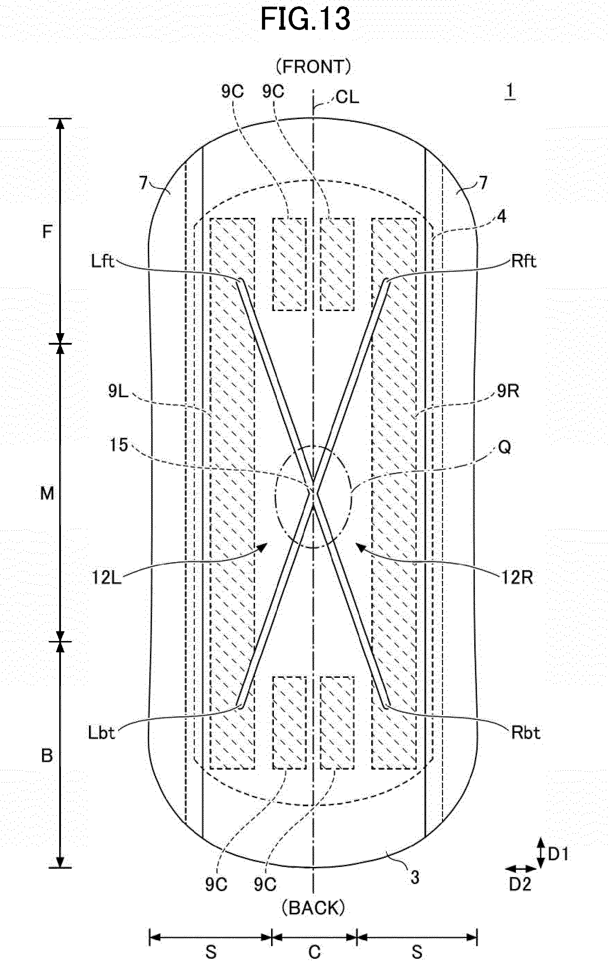

As a whole view, the example illustrated in Fig. 13 has a shape where a linear compressed groove extending from the front of the right side region S to the back of the left side region S and a linear compressed groove extending from the front of the left side region S to the back of the right side region S are crossed at the center of the left-right direction D2. Among the above-mentioned compressed grooves, the compressed grooves present at the left side of the front-back direction center line CL is determined as a left compressed groove 12L, and the compressed grooves present at the right side of the front-back direction center line CL is determined as a right compressed groove 12R. It may be also referred to as the left compressed groove 12L and the right compressed groove 12R being joined at the joint position 15.

The embodiments of the present invention have been described above, but the present invention is not limited to the above-described embodiments. Moreover, various modifications, changes, substitutions, additions, deletions, combinations, and the like may be made on the above-described embodiments within the scope defined by the claims, which are also included within the scope of the present invention.

Specific embodiments of the present invention will be described hereinafter.

### (Note 1)

In an embodiment of Note 1, an absorbent article includes a liquid-permeable top sheet, a liquid-impermeable back sheet, and an absorber disposed between the top sheet and the back sheet. The absorbent article has a front-back direction corresponding to a direction between a front and back of a body of a user, when the user wears the absorbent article, and a left-right direction orthogonal to the front-back direction. An anti-slip portion is disposed at a side of the absorbent article where the back sheet is disposed. One or more compressed grooves are formed at a side of the absorbent article where the top sheet is disposed. The one or more compressed grooves include a pair of a left compressed groove and a right compressed groove, both extending along the front-back direction to cross over a body-fluid-discharging-orifice counter portion in the front-back direction, where the body-fluid-discharging-orifice counter portion is an area of the absorbent article facing a body-fluid discharging orifice of the user when the user wears the absorbent article. The left compressed groove, as a whole, has a shape that curves towards right to pass through a central region of the left-right direction. The right compressed groove, as a whole, has a shape that curves towards left to pass through the central region of the left-right direction. In a planar view of the absorbent article, the left compressed groove and the right compressed groove do not overlap the anti-slip portion in the central region of the left-right direction, and a front end and a back end of the left compressed groove, and a front end and a back end of the right compressed groove overlap the anti-slip portion.

According to the embodiment of Note 1, the pair of the compressed grooves both extending along the front-back direction to cross over the body-fluid-discharging-orifice counter portion in the front-back direction, namely the left compressed groove and the right compressed groove, are formed at the side of the absorbent article where the top sheet is disposed. The left compressed groove has a shape that curves towards right to pass through the central region of the left-right direction, and the right compressed groove has a shape that curves towards left to pass through the central region of the left-right direction. As a force is applied to both sides of the absorbent article by legs of the user when the absorbent article is worn, the absorbent article can be deformed such that the central region of the left-right direction sinks towards the underwear side (non-skin side). Specifically, a groove-like dip can be formed along the front-back direction in a central region that includes the body-fluid-discharging-orifice counter portion at the center of the front-back direction. Since a discharged body fluid can be retained at least temporarily in such a dip, and the body fluid is guided along the front-back direction, leakage can be inhibited even when sudden discharge of a body fluid occurs or a large amount of a body fluid having a low viscosity is discharged. Moreover, at the front and back of the absorbent article, the pair of the left compressed groove and the right compressed groove can be respectively formed in the side regions that are present at the outer sides of the central region in the left-right direction. Therefore, the absorbent article can be brought into close contact with the front side and back side of the body without dipping of the area between the left compressed groove and the right compressed groove so that leakage can be prevented at the front and the back.

As described above, in a deformed state of an absorbent article that is induced by compressed grooves when a force is applied from legs, the front and back portions of the side regions of the left-right direction, i.e., the areas where the front ends and back ends of the left compressed groove and the right compressed groove are present, and the surrounding areas thereof, are particularly likely to be pulled in the direction towards the center of the front-back direction and the center of the left-right direction of the absorbent article (or towards the body-fluid-discharging-orifice counter portion). Therefore, the front and back of the side regions are prone to becoming detached from underwear so that fastening of the absorbent article to the underwear could potentially become unstable. According to the present embodiment, therefore, the front end and back end of the left compressed groove and the front end and back end of the right compressed groove overlap the anti-slip portion in a planar view. Thus, the fastening of the absorbent article onto underwear at the front and back of the side regions is more assuredly achieved so that the absorbent article is unlikely to detach from the underwear. Therefore, occurrences of wrinkling, twisting, or the like, which may be caused by an undesirable deformation of the absorbent article that is detached from the underwear, can be prevented. Therefore, a desired deformation state of the absorbent article that fits a body can be retained.

In the present embodiment, moreover, the left compressed groove and the right compressed groove do not overlap the anti-slip portion in the central region of the left-right direction in the planar view. Therefore, adhesion between the areas of the anti-slip portion to each other can be prevented even if the absorbent article is deformed to reduce the width of the central region when a force is applied from the left and right by legs. As a result, twisting of the absorbent article in the central region of the left-right direction can be prevented.

### (Note 2)

In an embodiment of Note 2, the left compressed groove and the right compressed groove are in contact with each other or overlap each other in the central region of the left-right direction.

According to the embodiment of Note 2, the left compressed groove and the right compressed groove are in contact with each other or overlap each other so that rigidity of the contact area or overlapped area is improved. Therefore, a dip towards an underwear side along the front-back direction can be assuredly formed in the middle region of the absorbent article in the central region of the left-right direction. Therefore, the above-described effect such that a body fluid is at least temporarily retained and is guided along the front-back direction is easily achieved. Moreover, the shape of the dip is stabilized once the dip is formed. Therefore, twisting or the like of the absorbent article can be prevented even if the absorbent article is worn over a long period.

### (Note 3)

In an embodiment of Note 3, the anti-slip portion includes at least a pair of a left anti-slip portion and a right anti-slip portion at both sides of the absorbent article, respectively. The left anti-slip portion and the right anti-slip portion are continuously formed along the front-back direction. The front end and the back end of the left compressed groove overlap the left anti-slip portion, and the front end and the back end of the right compressed groove overlap the right anti-slip portion.

According to the embodiment of Note 3, the anti-slip portion is continuously formed along the front-back direction in both side regions, thus the absorbent article can be more securely fastened along the front-back direction. Moreover, the left anti-slip portion constitutes the anti-slip portion that continues from the front end to the back end of the left compressed groove, and the right anti-slip portion constitutes the anti-slip portion that continues from the front end to the back end of the right compressed groove. Even in the case where the front or back of the absorbent article is twisted around the front-back direction center line as a central axis, therefore, the absorbent article is unlikely to detach from underwear so that fastening at a desired position and a desired deformation can be achieved.

### (Note 4)

In an embodiment of Note 4, a linear central compressed groove is formed along the front-back direction in the central region of the left-right direction. Part of the left compressed groove and part of the right compressed groove are included in the central compressed groove.

According to the embodiment of Note 4, a dip formed along the front-back direction, which sinks towards the underwear side, in the central region of the left-right direction is more easily formed by the linear central compressed groove. Therefore, the above-described effect such that a body fluid is at least temporarily retained and is guided along the front-back direction is easily achieved. Since part of the left compressed groove and part of the right compressed groove are included in the central compressed groove such that the front portions and back portions of both the left compressed groove and the right compressed groove are continuous with the central compressed groove, the deformation of the absorbent article is easily continuously formed along the front-back direction, and the deformation is likely to be retained stably.

### (Note 5)

In an embodiment of Note 5, in the planar view of the absorbent article, the left anti-slip portion and the right anti-slip portion are both spaced apart from the central compressed groove.

According to the embodiment of Note 5, a function of minimizing adhesion between the anti-slip portions in the central region of the left-right direction can be improved. As a result, the facilitation of the formation of the dip towards underwear by the central compressed groove can be enhanced.

### (Note 6)

In an embodiment of Note 6, front portions of the left compressed groove and the right compressed groove including the front end of the left compressed groove and the front end of the right compressed groove and/or back portions of the left compressed groove and the right compressed groove including the back end of the left compressed groove and the back end of the right compressed groove each have an arc shape that is convex towards a front-back direction center line extending along the front-back direction of the absorbent article.

According to the embodiment of Note 6, the absorbent article can be deformed more naturally to fit a body of a user at the front and/or back of the absorbent article.

This international application claims priority based on the Japanese patent application 2021-176393 filed on October 28, 2021, the entire contents of which are incorporated herein by reference.

### DESCRIPTION OF THE REFERENCE NUMERALS

- 1: absorbent article
- 2: back sheet
- 3: top sheet
- 4: absorber
- 7: side sheet
- 9C: central anti-slip portion
- 9L: left anti-slip portion
- 9R: right anti-slip portion
- 11: central compressed groove
- 12L: left compressed groove
- 12R: right compressed groove
- 12Lf: front portion of left compressed groove
- 12Lb: back portion of left compressed groove
- 12Rf: front portion of right compressed groove
- 12Rb: back portion of right compressed groove
- B: back region
- C: central region of left-right direction
- CL: center line of front-back direction
- D1: front-back direction (longitudinal direction)
- D2: left-right direction (width direction)
- F: front region
- M: middle region
- Lbt: back end of left compressed groove
- Lft: front end of left compressed groove
- Q: body-fluid-discharging-orifice counter portion
- Rbt: back end of right compressed groove
- Rft: front end of right compressed groove
- S: side region of left-right direction

## Claims

1. An absorbent article, comprising a liquid-permeable top sheet, a liquid-impermeable back sheet, and an absorber disposed between the top sheet and the back sheet,
wherein the absorbent article has a front-back direction corresponding to a direction between a front and back of a body of a user, when the user wears the absorbent article, and a left-right direction orthogonal to the front-back direction,
wherein an anti-slip portion is disposed at a side of the absorbent article where the back sheet is disposed,
wherein one or more compressed grooves are formed at a side of the absorbent article where the top sheet is disposed, the one or more compressed grooves including a pair of a left compressed groove and a right compressed groove, both extending along the front-back direction to cross over a body-fluid-discharging-orifice counter portion in the front-back direction, where the body-fluid-discharging-orifice counter portion is an area of the absorbent article facing a body-fluid discharging orifice of the user when the user wears the absorbent article,
wherein the left compressed groove, as a whole, has a shape that curves towards right to pass through a central region of the left-right direction, and the right compressed groove, as a whole, has a shape that curves towards left to pass through the central region of the left-right direction,
wherein, in a planar view of the absorbent article, the left compressed groove and the right compressed groove do not overlap the anti-slip portion in the central region of the left-right direction, and a front end and a back end of the left compressed groove, and a front end and a back end of the right compressed groove overlap the anti-slip portion.

2. The absorbent article according to claim 1,
wherein the left compressed groove and the right compressed groove are in contact with each other or overlap each other in the central region of the left-right direction.

3. The absorbent article according to claim 1 or 2,
wherein the anti-slip portion includes at least a pair of a left anti-slip portion and a right anti-slip portion at both sides of the absorbent article, respectively, the left anti-slip portion and the right anti-slip portion being continuously formed along the front-back direction, and
wherein the front end and the back end of the left compressed groove overlap the left anti-slip portion, and the front end and the back end of the right compressed groove overlap the right anti-slip portion.

4. The absorbent article according to claim 3,
wherein a linear central compressed groove is formed along the front-back direction in the central region of the left-right direction, and
wherein part of the left compressed groove and part of the right compressed groove are included in the central compressed groove.

5. The absorbent article according to claim 4, wherein, in the planar view of the absorbent article, the left anti-slip portion and the right anti-slip portion are both spaced apart from the central compressed groove.

6. The absorbent article according to any one of claims 1 to 5,
wherein front portions of the left compressed groove and the right compressed groove including the front end of the left compressed groove and the front end of the right compressed groove and/or back portions of the left compressed groove and the right compressed groove including the back end of the left compressed groove and the back end of the right compressed groove each have an arc shape that is convex towards a front-back direction center line extending along the front-back direction of the absorbent article.
